Europäisches Patentamt

European Patent Office

Office européen des brevets

(19)

(11) Publication number: **0 321 428**
A2

# (12) EUROPEAN PATENT APPLICATION

(21) Application number: 88850418.0

(22) Date of filing: 12.12.88

(51) Int. Cl.⁴: **A 61 K 31/08**

(30) Priority: **17.12.87 SE 8705035**

(43) Date of publication of application:
**21.06.89 Bulletin 89/25**

(84) Designated Contracting States:
**AT BE CH DE ES FR GB GR IT LI LU NL SE**

(71) Applicant: **HÄLSOPRODUKTER LARS KÄRNERUD AB**
**Box 145**
**S-57022 Forserum (SE)**

(72) Inventor: **Brohult, Sven**
**Gliavägen 97**
**S-161 52 Bromma (SE)**

**Brohult, Astrid**
**Gliavägen 97**
**S-161 52 Bromma (SE)**

(74) Representative: **Arwidi, Bengt**
**AHLPATENT AB Hemstigen 21**
**S-552 66 Jönköping (SE)**

The title of the invention has been amended (Guidelines for Examination in the EPO, A-III, 7.3).

(54) **Use of glycerol ethers in dermatological diseases.**

(57) This invention relates to the use of at least one glycerol ether as a means to affect diseases depending upon epidermal growth factor. The glycerol ethers are stable and can be orally administered in the form of capsules, which are brought on the market under the trademark ECOMER.

EP 0 321 428 A2

Bundesdruckerei Berlin

## Description

This invention relates to use of at least one glycerol ether for a pharmaceutical preparation. The pharmaceutical preparation is for treatment of diseases and effects depending upon epidermal growth. It has been found that the pharmaceutical preparation has beneficial influence on the so-called epidermal growth factor.

Glycerol ethers (previously also called alcoxy-glycerols) have shown several important medical effects. Oral administration of glycerol ethers by radiation therapy reduces the number of harmful radiation effects; leucopenia and trombocytopenia is partly or fully prevented. By certain tumor diseases a lower mortality is obtained in a group which has been given glycerol ethers as profylactic treatment as compared to a group which was not given these substances. Experiments both with humans and animals have shown that the glycerol ethers improve the bodily immunity defense mechanism.

In a human body glycerol ethers are found mainly in bone marrow, liver, mother's milk and placenta. The glycerol ethers are found in larger quantities mainly in shark liver oil.

The glycerol ethers have the following general formula:

$CH_2 . OH$

$CH . OH$

$CH_2 . O . R,$

wherein R represents a longchained, alihpatic hydrocarbon chain. Naturally occurring glycerol ethers are mixtures with varying number of carbon atoms in the side chain. Compounds having 16 or 18 carbon atoms make up most the quantities, but the number of carbon atoms varies mainly with the interval of 14-24. E.g. chimmyl alcohol and batyl alcohol are saturated glycerol ethers having 16 and 18 carbon atoms respectively in the side chain. Selachyl alcohol is an unsaturated glycerol ether with 18 carbon atoms. There also are found methoxysubstituted glycerol ethers, i.e. compounds wherein one hydrogen atom, mostly on carbon atom 2, has been replaced by a methoxy group ($-OCH_3$).

In the human body like in shark liver oil the glycerol ethers are present as di-esters of fatty acids. They differ from ordinary fat (triglycerid, i.e. a tri-ester of fatty acids) only at one point in the molecule, namely that one of the three ester bonds is replaced by an ether bond. Surprisingly, it has been found, that the glycerol ethers beneficially influence diseases depending upon epidermal growth. It has been found that severe wounds heal, eczema disappears fully or partly and certain cases of psoriasis are much improved. Further, in some cases, severe haemrnorhoids healed after 1-2 months of oral administration of glycerol ethers. Burns caused by intensive sunray can be partly prevented.

Psoriasis is a skin disease which strikes about 2% of the population. The formation of new epidermal cells increases while the wearing out remains unchanged. Disfiguring, strongly reddening raised areas or spots are formed, which are covered by thick scale. Psoriasis mainly appears on elbows and knees and in the loin area but may appear at any place of the body. Hands and feet may have raised areas with thick skin, which may cause hurting cracks and bothering itching often occurs. The disease is not catching and it has now been found that it may be fully or partly healed by therapy using glycerol ethers.

Psoriasis and other diseases mentioned above have previously been treated with cortisone ointment. Such treatment, however, only has local and shortterm effects. The glycerol ethers may be orally administrated and are stable compounds. Therefoe they have long-term systemic effects, like the effects previously found from certain proteins, which are present in very small amounts in the body and affects the epidermal growth. These proteins, however, are unstable and active only for a short time.

In the following examples glycerol ethers have been administered orally using capsules, which are available under the trademark ECOMER. Each capsule contains 0.05 g of the active substance, which comprises a mixture of glycerol ethers and is produced from shark liver.

In the following examples results are given from therapy with ECOMER in some cases of psoriasis of various degree of severity.

Example 1

A patient with very severe psoriasis, where the reddening areas with spots of thick scale were spread all over the body, had been given a therapy with cortison ointments without any obvious result. When the therapy with glycerol ethers was started the disease had been going on for about 35 years. This patient was administered glycerol ethers in the form of 12 capsules of ECOMER per day. After a little more than 1 month an obvious improvement was observed and after a little more than another month the disease was gone. After another 4 months the improvement was fully maintained.

Example 2

4 patients, having not so severe psoriasis as of example 1, have each been administered glycerol ethers in the form of 6 capsules of ECOMER per day. Improvements were observed after 2 to 3 months. The itching irritation caused by the disease was gone and the reddening, raised areas had disappeared from large parts of the body.

Example 3

In this group 15 patients have been administered glycerol ethers in the form of 2-4 capsules of ECOMER per day. In these cases the irritation has ceased and by some of the patients the reddening areas have been reduced.

## Claims

1) Use of at least one glycerol ether for making a pharmaceutical preparation for treatment of diseases and wounds or injuries depending upon epidermal growth.

2) Use according to claim 1, **characterized** in that the mixture of glycerol ethers being present in shark liver oil is used.

3) Use according to claim 1, **characterized** in that one or more glycerol ethers with a straight side chain is used.

4) Use according to claim 1, **characterized** in tht one or more methoxy-substituted glycerol ethers is used.

5) Use according to claim 1, **characterized** in that a mixture of glycerol ethers having straight side chains and methoxy-substituted glycerol ethers is used.